(19) Europäisches Patentamt
European Patent Office
Office européen des brevets

(11) **EP 1 679 508 B1**

(12) **EUROPEAN PATENT SPECIFICATION**

(45) Date of publication and mention
of the grant of the patent:
**21.07.2010 Bulletin 2010/29**

(21) Application number: **04793193.6**

(22) Date of filing: **29.10.2004**

(51) Int Cl.:
***G01N 27/327*** [(2006.01)]     ***C12Q 1/00*** [(2006.01)]

(86) International application number:
**PCT/JP2004/016085**

(87) International publication number:
**WO 2005/043146 (12.05.2005 Gazette 2005/19)**

(54) **BIOSENSOR AND METHOD FOR PREPARATION THEREOF**

BIOSENSOR UND VERFAHREN ZU DESSEN HERSTELLUNG

BIOCAPTEUR ET PROCEDE DE FABRICATION ASSOCIE

(84) Designated Contracting States:
**AT BE BG CH CY CZ DE DK EE ES FI FR GB GR HU IE IT LI LU MC NL PL PT RO SE SI SK TR**
Designated Extension States:
**AL HR LT LV MK**

(30) Priority: **30.10.2003 JP 2003371198**

(43) Date of publication of application:
**12.07.2006 Bulletin 2006/28**

(73) Proprietor: **ARKRAY, Inc.**
**Kyoto-shi, Kyoto 601-8045 (JP)**

(72) Inventors:
• **YAMAOKA, Hideaki**
**Kyoto (JP)**
• **TSUJIMOTO, Tomomichi**
**Kyoto (JP)**

(74) Representative: **Matthews, Derek Peter**
**Dehns**
**St Bride's House**
**10 Salisbury Square**
**London**
**EC4Y 8JD (GB)**

(56) References cited:
**EP-A- 0 884 392        EP-A- 1 235 068**
**EP-A- 1 336 839        WO-A1-01/36954**
**WO-A1-02/35222       JP-A- 2 231 558**
**JP-A- 11 002 618**

• **OHSAKA, T. ET AL.: 'A New Amperometric Glucose Sensor Based on Bilayer Film Coating of Redox-Active Clay Film and Glucose Oxidase Enzyme Film' BULLETIN OF THE CHEMICAL SOCIETY OF JAPAN vol. 63, no. 9, 1990, pages 2646 - 2652, XP002983672**
• **COSNIER, S. ET AL.: 'A new strategy for the construction of amperometric dehydrogenase electrodes based on laponite gel-methylene blue polymer as the host matrix' JOURNAL OF ELECTROANALYTICAL CHEMISTRY vol. 406, no. 1, 1996, pages 243 - 246, XP002983673**
• **ZEN, J.M. ET AL.: 'An Enzymatic Clay-Modified Electrode for Aerobic Glucose Monitoring with Dopamine as Mediator' ANALYTICAL CHEMISTRY vol. 69, no. 8, 1997, pages 1669 - 1773, XP000690170**
• **ZEN, J.M. ET AL.: 'A Glucose Sensor Made of an Enzymatic Clay-Modified Electrode and Methyl Viologen Mediator' ANALYTICAL CHEMISTRY vol. 68, no. 15, 1996, pages 2635 - 2640, XP000623885**
• **POYARD, S. ET AL.: 'A new method for the controlled immobilization of enzyme in inorganic gels (laponite) for amperometric glucose biosensing' SENSORS AND ACTUATORS B vol. 33, no. 1, 1996, pages 44 - 49, XP000632923**

EP 1 679 508 B1

**Description**

Technical Field

The present invention relates to a biosensor for electrochemically measuring an analyte in a sample.

Background Art

Biosensors that can quantify a specific analyte in a sample solution simply and rapidly, for example, without diluting or stirring the sample solution have been used widely. Such a biosensor can be produced by, for example, forming an electrode system having a working electrode (also referred to as "measuring electrode") and a counter electrode on an electrically insulating substrate by a method such as screen printing, and forming a reagent layer including an oxidoreductase that reacts with the analyte and a mediator (an electron carrier) on the electrode system (see, Patent Documents 1-4, for example). When the reagent layer is in contact with the sample solution containing the analyte, the analyte is oxidized and the mediator is reduced by the catalytic action of the oxidoreductase, for example. The mediator thus reduced (hereinafter referred to as "reduced mediator") is reoxidized electrochemically using the electrode system, and the concentration of the analyte in the sample solution can be calculated from the thus-obtained oxidation current value.

However, in the case where oxygen is present in the reaction atmosphere or the sample solution contains dissolved oxygen, the reduced mediator is not only electrochemically reoxidized as described above but also reoxidized by the oxygen. Accordingly, there has been a problem in that an error may be caused in the oxidation current value obtained through the electrochemical reoxidation, so that the measurement accuracy is deteriorated.

As a method for avoiding such a problem, carrying out the measurement in a nitrogen atmosphere may be considered, for example. However, this takes more time and complicates the operation. Furthermore, under the conditions completely free from oxygen, oxidoreductases that require oxygen to cause an enzyme reaction cannot be used. This makes the applicable range of the biosensor very small.

Patent Document 1: JP 1(1989)-291153 A
Patent Document 2: EP-A-0 884 392
Patent Document 3: EP-A-1 235 068
Patent Document 4: EP-A-1 336 839

Disclosure of Invention

Problem to be Solved by the Invention

With the foregoing in mind, it is an object of the present invention to provide a biosensor that can prevent a mediator from being affected by oxygen, thereby allowing an analyte in a sample solution to be measured rapidly and easily with high accuracy.

Means for Solving Problem

In order to achieve the above object, a method for producing a biosensor of the present invention includes: providing a substrate having an electrode; and forming an inorganic gel layer that contains at least a mediator, an ampholytic surfactant, a buffer, and a layered inorganic compound on a surface of the electrode.

Furthermore, a biosensor of the present invention is a biosensor produced by the above-described method of the present invention.

Effects of the Invention

The inventors of the present invention conducted keen studies with a view to providing a biosensor capable of preventing the natural oxidation of a mediator (reduced mediator) that has been reduced by the reaction between an analyte in a sample and an oxidoreductase. As a result, the inventors of the present invention found that, when an inorganic gel layer, which had been generally known to be formed using a layered inorganic compound, further contained a surfactant and a buffer, the inorganic gel layer could prevent natural oxidation of the reduced mediator. It should be noted that the inventors of the present invention were the first to find that the effect of preventing the natural oxidation could be obtained by this approach. The inorganic gel layer as described above can prevent the reduced mediator for indirectly measuring an analyte in a sample from being reoxidized by, for example, oxygen present in the measurement atmosphere, dissolved oxygen in the sample, or the like. Thus, it becomes possible to provide a biosensor that remedies the measurement error caused by the reoxidation of the reduced mediator and thus achieves excellent measurement accuracy. It is to be noted that, in the present invention, "natural oxidation of the mediator" refers to oxidation of the mediator caused during the use of a biosensor by, for example, dissolved oxygen in a liquid sample or oxygen that has been absorbed in moisture in the air (moisture in the air absorbs oxygen, for example, during the storage of the biosensor).

Presumably, through the following mechanism, the oxidation-preventing function is obtained by forming an inorganic gel layer in the presence of a surfactant and a buffer.

For example, in an inorganic gel layer formed by applying a dispersion containing a mediator, an ampholytic surfactant, a buffer, and a layered inorganic compound, it is presumed that the mediator is intercalated firmly between sheets of the layered inorganic compound to form a composite, and this allows the mediator to be prevented from being brought into contact with dissolved oxygen contained in a liquid sample or the like. Sample solutions generally contain dissolved oxygen. Thus, the mediator can be prevented from being affected by the dissolved oxygen when water is blocked by the layered inorganic compound as described above. The presence of the surfactant in the inorganic gel layer as described above prevents insolubilization of the layered inorganic compound and the mediator due to their aggregation from occurring. Thus, the composite of the layered inorganic compound and the mediator is dispersed, so that the inorganic gel layer capable of sufficiently producing the above-described effect can be obtained.

Furthermore, when the dispersion contains a buffer, a uniform inorganic gel layer can be formed so that the oxidation-preventing function can be improved further. The reason for this is considered to be that the buffer acts as a binder when the composite of the mediator and the layered inorganic compound is formed. Due to the action of the buffer as a binder, a composite in which the mediator is bound to the layered inorganic compound still more firmly can be formed, so that, for example, a liquid containing dissolved oxygen can be blocked still more effectively to prevent the reoxidation of the mediator by oxygen. In this case, it is considered that the ampholytic surfactant also acts as a so-called blocking agent for preventing the buffer, the layered inorganic compound, and the mediator from being indispersible due to their aggregation.

A biosensor of the present invention produced by the above-described method can prevent the mediator from being reoxidized by oxygen present in the measurement atmosphere, dissolved oxygen in the sample, or the like, for example. Accordingly, the biosensor of the present invention can remedy the measurement error caused by the reoxidation of the reduced mediator and thus can achieve excellent measurement accuracy.

In the biosensor produced by the method of the present invention, it is considered that electron transfer from the oxidoreductase to the mediator is achieved via an interlayer of the layered inorganic compound, i.e., an electric double layer, rather than via water. Thus, according to the biosensor of the present invention in which moisture is blocked by the inorganic gel layer, an advantageous effect that the biosensor is prevented from being degraded under high humidity conditions or the like can be obtained, for example. Moreover, electrons pass through the electric double layer of the layered inorganic compound more easily than they pass through water, which can lead to an increase in reaction velocity.

Furthermore, according to the biosensor produced by the method of the present invention, the formation of rust on the electrode due to oxidation, for example, also can be prevented by the above-described blocking of oxygen.

In the present invention, the inorganic gel layer for preventing natural oxidation of the mediator also is referred to as the "oxidation-preventing layer".

Brief Description of Drawings

[FIG. 1] FIG. 1 shows one example of a method for producing a biosensor in an embodiment of the present invention, wherein FIGs. 1A to 1F respectively show major steps in the method.

[FIG. 2] FIG. 2 is a sectional view of the biosensor in the above embodiment.

[FIG. 3] FIG. 3 is a graph showing the change in measured current value over time when a sample is measured using a glucose sensor in an example of the present invention, wherein FIG. 3A shows the results obtained in the example and FIG. 3B shows the results obtained in a comparative example.

[FIG. 4] FIG. 4 is a graph showing the relationship between the concentration of dissolved oxygen in a sample and the rate of change (%) in another example of the present invention.

[FIG. 5] FIG. 5 is a graph showing the relationship between the concentration of dissolved oxygen in a sample and the rate of change (%) in still another example of the present invention.

[FIG. 6] FIG. 6 is a graph showing the relationship between the concentration of dissolved oxygen in a sample and the rate of change (%) in still another example of the present invention.

Description of the Invention

In the present invention, the layered inorganic compound preferably is a layered clay mineral, particularly preferably an expansive (swelling) clay mineral.

The layered inorganic compound refers to, for example, an inorganic compound in which polyhedrons of an inorganic substance are linked horizontally to form a sheet structure and a plurality of such sheet structures are laminated to form a layered crystal structure. The polyhedron may be, for example, a tetrahedron or an octahedron, more specifically, an Si tetrahedron or an Al octahedron. Examples of such a layered inorganic compound include layered clay minerals, hydrotalcite, smectite, halloysite, kaolin minerals, and mica.

In general, a layered clay mineral refers to an aluminum silicate mineral, which forms the most part of clay (the clay is fine-grained soil that is plastic when wetted with water). In general, the minimum constitutional unit of the layered clay mineral is a Si tetrahedron that is composed of Si surrounded by four oxygen atoms (O), an Al octahedron that is composed of Al surrounded by six hydroxyl groups (OH groups) or six oxygen atoms, or a Mg octahedron that is composed of Mg surrounded by six hydroxyl groups (OH groups) or six oxygen atoms.

The layered clay mineral has a layered structure in which adjoining Si tetrahedrons share one plane and the remaining apical oxygen atoms are directed in the same direction to form a sheet with a hexagonal net-like pattern (hereinafter referred to as a "tetrahedral sheet"), adjoining Al or Mg octahedrons share an edge to form a sheet (an octahedral sheet), and a plurality of such tetrahedral sheets and octahedral sheets are laminated, for example. More specifically, minerals having a structure in which a plurality of 1:1 layers, each composed of one tetrahedral sheet and one octahedral sheet, are laminated are called 1:1 minerals; minerals having a structure in which a plurality of 2:1 layers, each composed of two tetrahedral sheets and one octahedral sheet sandwiched therebetween, are called 2:1 minerals; and minerals having a structure in which each of the 2:1 layers includes one more octahedral sheet between the tetrahedral sheets are called 2:1:1 minerals, for example. Furthermore, minerals having a structure in which the octahedral sheets are $Mg(OH)_2$ sheets and metal ions are at all the possible sites of the octahedrons are called trioctahedral minerals, and minerals having a structure in which the octahedral sheets are Al $(OH)_3$ sheets and 1/3 of the Al $(OH)_3$ sheets are vacant are called dioctahedral minerals. Among these, 2:1 minerals are preferable as the layered inorganic compound to be used in the present invention.

Examples of an element composing the layered inorganic compound include lithium, sodium, potassium, magnesium, aluminum, silicon, oxygen, hydrogen, fluorine, and carbon. The layered inorganic compound may be composed of only one element or two or more elements. Specific examples of the layered inorganic compound include those represented by the following formulae (1) to (9), though it is to be noted that the layered inorganic compound is not particularly limited to these examples. These compounds may contain crystal water, for example. Note here that, although the following formulae represent mineralogically or chemically pure compounds, these compounds may in fact contain impurities such as sodium silicate, for example. Thus, the chemical formulae of these compounds determined by elemental analysis or the like are not always identical to the following formulae. This is mentioned in the literature (e.g., D. W, Thompson, J. T. Butterworth, J. Colloid Interf. Sci., 151, 236-243 (1992)).

$$M_x Si4 (Al_{2-x}Mg_x)O_{10}X_2 \qquad (1)$$

In the above formula (1), it is preferable that: M is at least one selected from the group consisting of H, Li, Na, and K; X is at least one of OH and F; and $_x$ is a positive number of less than 2.

$$M_x(Si_{4-x}Al_x)Al_{12}O_{10}X_2 \qquad (2)$$

In the above formula (2), it is preferable that: M is at least one selected from the group consisting of H, Li, Na, and K; X is at least one of OH and F; and is a positive number of less than 4.

$$M_x Si_4(Mg_{3-x}Li_x)O_{10}X_2 \qquad (3)$$

In the above formula (3), it is preferable that: M is at least one selected from the group consisting of H, Li, Na, and K; X is at least one of OH and F; and $_x$ is a positive number of less than 3.

$$M_x(Si_{4-x}Al_x)Mg_3O_{10}X_2 \qquad (4)$$

In the above formula (4), it is preferable that: M is at least one selected from the group consisting of H, Li, Na, and K; X is at least one of OH and F; and $_x$ is a positive number of less than 4.

$$MSi_4Mg_{2.5}O_{10}X_2 \qquad (5)$$

In the above formula (5), M preferably is at least one of Li and Na, more preferably is Na; and X preferably is at least one of OH and F, more preferably is F.

$$MzSi_4Mg_2O_{10}X_2 \qquad (6)$$

In the above formula (6), M preferably is at least one of Li and Na, more preferably is Li; and X preferably is at least one of OH and F, more preferably is F.

$$Mg_6Al_{12}(OH)_{16}X_x \qquad (7)$$

In the above formula (7), X preferably is at least one of an anionic organic acid and at least one group selected from the group consisting halogen, $NO_3$, $SO_4$, $CO_3$, and OH, more preferably is $CO_3$; and it is preferable that, when X is halogen, OH, $NO_3$ or a monovalent organic acid, $x$ is 2, and, when X is $SO_4$, $CO_3$ or a divalent organic acid, $x$ is 1.

$$Na_{0.33}Si_4(Mg_{2.67}Li_{0.33})O_{10}X_2 \qquad (8)$$

In the above formula (8), X preferably is at least one of OH and F, more preferably is OH.

$$Na_{a-b}(Si_{4-a}Al_a)(Mg_{3-b}Al_b)O_{10}X_2 \qquad (9)$$

In the above formula (9), X preferably is at least one of OH and F, more preferably is OH; a preferably is a positive number of less than 4; $b$ preferably is a positive number of less than 3; and a-b > 0 preferably is satisfied.
Specific examples of the layered inorganic compound include: 1:1 clay minerals such as kaolinite, halloysite, and serpentine; 2:1 clay minerals such as talc; pyrophyllite, smectite, vermiculite (represented by the above formula (2), hereinafter the same), tetrasilicic fluorine mica (the above formula (5)), and mica containing taeniolite (the above formula (6)); 2:1:1 clay minerals such as chlorite; minerals intermediate between 2:1 clay minerals and 2:1:1 clay minerals; subcrystalline clay minerals such as imogolite; amorphous clay minerals such as allophane; and hydrotalcite (the above formula (7)).
Smectite is divided into several species according to the type of ion that is present in the lattices of tetrahedrons and octahedrons as a result of isomorphous replacement. Examples of the species of smectite include: dioctahedral smectite such as montmorillonite (the above formula (1)), bentonite as a natural product containing 40% to 80% of montmorillonite, and beidellite (the above formula (2)); and trioctahedral smectite such as hectorite (the above formula (3), preferably the above formula (8)), saponite (the above formula (4), preferably the above formula (9)), and nontronite.
Hydrotalcite is represented by, for example, the above formula (7). More specifically, hydrotalcite is a layered mineral represented by, for example, $Mg_6Al_2(OH)_{16}CO_3 \cdot 4H_2O$, where a part of $Mg^{2+}$ in $Mg(OH)_2$ (brucite: brucite has a structure in which a plurality of oxygen octahedron sheets having $Mg^{2+}$ in their center are laminated) is isomorphously replaced by $Al^{3+}$. Although $Al^{3+}$ is positively charged, hydrotalcite maintains electrical neutrality due to $CO_3^{2-}$ present in an interlayer and thus has an anion exchange capacity. Although hydrotalcite is not a silicate mineral, it generally is treated as a clay mineral.
Examples of the compositions of the above-described layered inorganic compounds are shown in Table 1 below. In Table 1, "MI" denotes an exchangeable cation represented as a monovalent cation, e.g., $H^+$, $Na^+$, $K^+$ or $Li^+$.
[Table 1]

| Name of minerals | Composition |
|---|---|
| Kaolinite | $Si_2Al_2O_5(OH)_4$ |
| Halloysite | $Si_2M_2O_5(OH)_4 \cdot 2H_2O$ |
| Serpentine | $Si_2(Mg^{2+},Fe^{2+})_3O_5(OH)_4$ |
| Talc | $Si_4Mg_3(OH)_2O_{10}$ |
| Pyrophyllite | $Si_4Al_2(OH)_2O_{10}$ |
| Montmorillonite | $MI_xSi_4(Al_{2-x}Mg_x)O_{10}(OH)_2 \cdot nH_2O$ |
| Beidellite | $MI_x(Si_{4-x}Al_x)Al_2O_{10}(OH)_2 \cdot nH_2O$ |
| Hectorite | $MI_xSi_4(Mg_{3-x}Li_x)O_{10}(OH,F)_2 \cdot nH_2O$ |
| Saponite | $MI_x(Si_{4-x}Al_x)Mg_3O_{10}(0H)_2 \cdot nH_2O$ |
| Nontronite | $MI_x(Si_{4-x}Al_x)Fe_2O_{10}(OH)_2 \cdot nH_2O$ |

(continued)

| Name of minerals | Composition |
| --- | --- |
| Vermiculite | $MI_x(Si_{4-x}Al_x)Al_2O_{10}(OH)_2 \cdot nH_2O$ |
| Hydrotalcite | $Mg_6Al_2(OH)_{16}CO_3 \cdot 4H_2O$ |

The average particle diameter of the layered inorganic compound is not particularly limited, but it preferably is such that it allows the layered inorganic compound to be dispersed in a solvent uniformly. In general, the layered inorganic compound is composed of plate-like particles, which are in dynamic equilibrium where some of the particles repeat aggregation and cleavage. Thus, defining the average particle diameter itself is difficult. However, the average particle diameter measured by, for example, light scattering method or observation using an electron microscope in the state where the layered inorganic compound is dispersed in water preferably is in the range from 1 nm to 20 $\mu$m, more preferably from 10 nm to 2 $\mu$m.

In the above-described various layered inorganic compounds such as clay minerals, the distance between adjacent sheets and the electric charge or the polarity of an interlayer can be adjusted beforehand by the use of pillar such as quaternary ammonium salt, for example.

Among the above-described layered inorganic compounds, 2:1 clay minerals are more preferable, and expansive clay minerals having an ion exchange capacity are particularly preferable.

Among the above-described expansive clay minerals, bentonite, smectite, vermiculite, synthesized fluorine mica, and the like are more preferable, and synthesized smectite such as synthesized hectorite and synthesized saponite, expansive synthesized mica typified by synthesized fluorine mica, and synthesized mica such as Na-mica (note here that natural mica generally is inexpansive clay mineral) are particularly preferable. These layered inorganic compounds may be used alone or in combination of at least two kinds thereof.

As the layered inorganic compound, it is possible to use a commercially available product such as products named "LUCENTITE SWN", "LUCENTITE SWF" (synthesized hectorite), and "ME" (fluorine mica) manufactured by CO-OP CHEMICAL Co. Ltd., a product named "SUMECTON SA" (synthesized saponite) manufactured by Kunimine Industries, Co. Ltd., products named "THIXOPY W" (synthesized hectorite) and "KYOWAAD 500" (synthesized hydrotalcite) manufactured by Kyowa Chemical Industry Co., Ltd., a product named "Laponite" (synthesized hectorite) manufactured by Laporte Industries Ltd., natural bentonite available from Nacalai Tesque, Inc., a product named "Multigel" (bentonite) manufactured by Hojun Kogyo Co., Ltd., or the like.

In the present invention, the surfactant is an ampholytic surfactant, more preferably an ampholytic surfactant having a positive charge and a negative charge in a single molecule, e.g., alkylaminocarboxylic acid (or a salt thereof), a carboxybetaine, a sulfobetaine, or a phosphobetaine. Among these, an ampholytic surfactant having a positive charge and a negative charge that are separated from each other in a single molecule is still more preferable. Examples of such an ampholytic surfactant include carboxybetaines, sulfobetaines, and phosphobetaines. More specifically, alkyldimethylamino acetic acid betaine or the like can be used as the carboxybetaine, and CHAPS, CHAPSO, alkyl hydroxysulfobetaine, or the like can be used as the sulfobetaine. Among these, sulfobetaines are preferable, CHAPS and CHAPSO are more preferable, and CHAPS is particularly preferable.

In the present invention, the buffer preferably is an amine buffer. Examples of the amine buffer include Tris, ACES, CHES, CAPSO, TAPS, CAPS, Bis-Tris, TAPSO, TES, Tricine, and ADA. Among these, ACES and Tris are preferable, and ACES is more preferable. These substances may be used alone or in combination of at least two kinds thereof.

As the buffer, a buffer having a carboxyl group also is preferable. Examples the buffer having a carboxyl group include an acetic acid-sodium acetate buffer, a malic acid-sodium acetate buffer, a malonic acid-sodium acetate buffer, and a succinic acid-sodium acetate buffer. Among these, a succinic acid-sodium acetate buffer is preferable.

Examples of the combination of the amine buffer and the surfactant include the combinations of Tris and CHAPS, ACES and CHAPS, and ACES and CHAPSO. Among these, the combination of CHAPS and ACES is more preferable. Furthermore, examples of the combination of the buffer having a carboxyl group and the surfactant includes the combinations of succinic acid-sodium acetate and CHAPS or CHAPSO, malonic acid-sodium acetate and CHAPS, malic acid-sodium acetate and CHAPS or CHAPSO, and acetic acid-sodium acetate and CHAPS. Among these, the combination of succinic acid-sodium acetate and CHAPS is preferable.

In the present invention, it is preferable that the mediator is, for example, a mediator that turns into a reduced mediator by the reaction between an oxidoreductase and an analyte that will be described later, so that it is oxidized electrochemically and detected by detecting an oxidation current. Conventionally known mediators can be used as the mediator.

Specific examples of the mediator include potassium ferricyanide, p-benzoquinone and derivatives thereof, phenazine methosulfate, indophenol, indophenol derivatives such as 2,6-dichloro phenol indophenol, β-naphthoquinone-4-sulfonic acid potassium salt, ferrocene, ferrocene derivatives such as ferrocenecarboxylic acid, osmium complexes, ruthenium complexes, $NAD^+$, $NADP^+$, pyrrolo-quinoline quinine (PQQ), methylene blue, cytochtome c, cytochrome b, and copper complexes. Among these, potassium ferricyanide, ferrocene, osmium complexes, ruthenium complexes, $NAD^+$, $NADP^+$, and the like are preferable.

Other than the above, the following substances also can be used as the mediator, for example: 1,1'-dimethyl-4,4'-bipyridinium salt, 1,1'-dibenzyl-4,4'-bipyridinium salt, 1,4-diaminobenzene, 2-methyl-1,4-naphthoquinone, N-methylphenazinium salt, 1-hydroxy-5-methylphenazinium salt, 1-methoxy-5-methylphenazinium salt, 9-dimethylaminobenzo alpha phenoxazine-7-ium salt, hexacyanoferrate (II), 7-hydroxy-3H-phenoxazine-3-one 10-oxide, 3,7-diamino-5-phenylphenazinium salt, 3-(diethylamino)-7-amino-5-phenylphenazinium salt, 1,4-benzendiol, 1,4-dihydroxy.2,3,5-trimethylbenzene, N,N,N',N'-tetramethyl-1,4-benzenediamine, $\Delta$2,2'-bi-1,3-dithiol, 2,6-dimethylbenzoquinone, 2,5-dimethylbenzoquinone, 2,3,5,6-tetramethyl-2,5-cyclohexadiene-1,4-dione, 2,6-dichloro-4-[(4-hydroxyphenyl)imino]-2,5-cyclohexadiene-1-one, 2,6-dichloro-4-[(3-chloro-4-hydroxyphenyl)imino]-2,5-cyclohexadiene-1-one, 7-(diethylamino)-3-imino-8-methyl-3H-phenoxazine salt, and 3,7-bis(dimethylamino) phenothiazine-5-ium salt.

In the method of the present invention, it is preferable that the inorganic gel layer is formed by applying a dispersion containing at least a mediator, a surfactant, a buffer, and a layered inorganic compound.

In the present invention, a reagent layer as a laminate may be formed by forming a layer containing an oxidoreductase on the inorganic gel layer. Alternatively, by using the dispersion that further contains an oxidoreductase, a reagent layer as a single layer may be formed by forming an inorganic gel layer containing the oxidoreductase on the electrode surface. When the reagent layer as a single layer is formed as described above, it is not necessary to form a layer containing an oxidoreductase and a layer containing a mediator separately, so that the biosensor can be produced still more easily. Such a biosensor is particularly preferable, for example, when using an enzyme that does not require oxygen to cause an enzyme reaction, such as glucose dehydrogenase (GDH).

The oxidoreductase is not particularly limited, for example, as long as it causes a redox reaction with an analyte in a sample and with the mediator, and may be determined as appropriate depending on the type of the analyte.

Specific examples of the oxidoreductase include glucose oxidase (GOD), pyranose oxidase, glucose dehydrogenase (GDH), lactate oxidase, lactate dehydrogenase, fructose dehydrogenase, galactose oxidase, cholesterol oxidase, cholesterol dehydrogenase, alcohol oxidase, alcohol dehydrogenase, bilirubin oxidase, glucose-6-phosphate dehydrogenase, amino-acid dehydrogenase, formate dehydrogenase, glycerol dehydrogenase, acyl-CoA oxidase, choline oxidase, 4-hydroxybenzoic acid hydroxylase, maleate dehydrogenase, sarcosine oxidase, and uricase.

The combination of the oxidoreductase and the mediator is not particularly limited, and examples thereof include the combinations of GOD and potassium ferricyanide, GDH and a ruthenium complex, cholesterol dehydrogenase and ferrocene, and alcohol dehydrogenase and a copper complex.

(Embodiment 1)

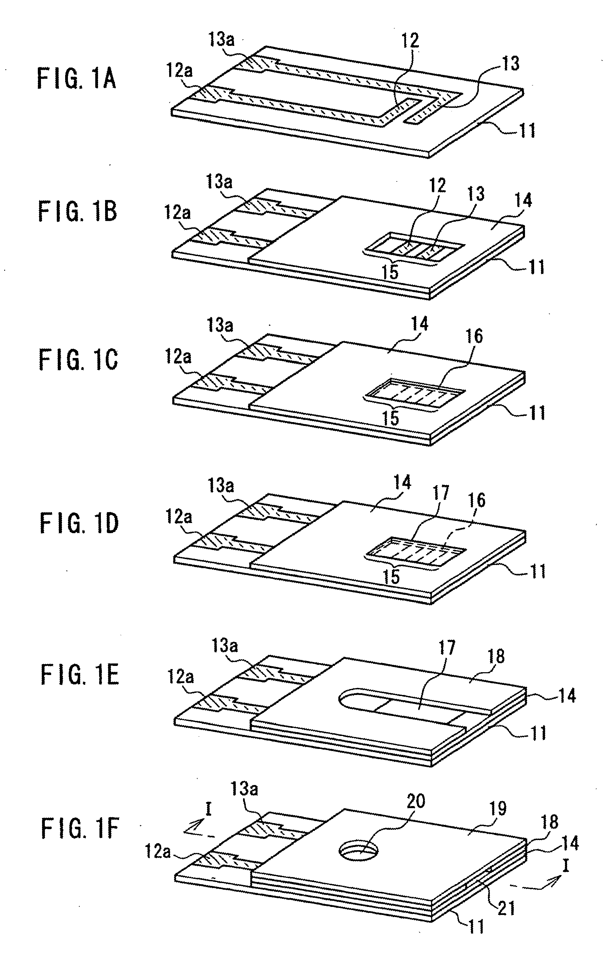

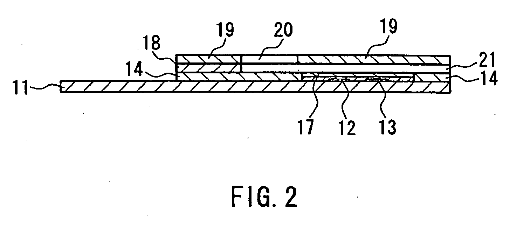

An example of a first method for producing a biosensor according to the present invention will be described with reference to FIG. 1 and FIG. 2. FIGs. 1A to 1F are perspective views showing a series of major steps in the production of a biosensor. FIG. 2 is a sectional view of the biosensor taken in the arrow direction of line I-I in FIG. 1F. In FIGs. 1A to 1F and FIG. 2, the same components are given the same reference numerals.

As shown in FIG. 1F and FIG. 2, this biosensor 1 includes: a substrate 11; an electrode system including a working electrode 12 having a lead portion 12a and a counter electrode 13 having a lead portion 13a; an insulating layer 14; an inorganic gel layer (oxidation-preventing layer) 16 containing a mediator, a layered inorganic compound, and a surfactant; an enzyme reagent layer 17 containing an oxidoreductase; a spacer 18 having an opening; and a cover 19 having a through hole 20. As shown in FIG. 1B, a detecting portion 15 is provided on one end portion (on the right in FIGs. 1 and 2) of the substrate 11, and in the detecting portion 15, the working electrode 12 and the counter electrode 13 extend in the width direction of the substrate 11 so as to be parallel to each other. One end of the working electrode 12 and one end of the counter electrode 13 serve as the lead portion 12a and the lead portion 13a, respectively (on the left in FIGs. 1 and 2), which are orthogonal to the other ends of the respective electrodes in the detecting portion 15 (FIG. 1A). A portion between the working electrode 12 and the counter electrode 13 is an insulating portion. As shown in FIG. 1B, the insulating layer 14 is laminated on the substrate 11 having the electrode system with such a configuration, except on the lead portions 12a and 13a and the detecting portion 15. On the detecting portion 15 on which the insulating layer 14 is not laminated, the inorganic gel layer 16 and the enzyme reagent layer 17 are laminated in this order. On the insulating layer 14, the spacer 18 having an opening at a portion corresponding to the detecting portion 15 is disposed, as shown in FIG. 1E. On the spacer 18, the cover 19 having the through hole 20 at a part of the portion corresponding to the opening is disposed (FIG. 1F). In this biosensor 1, a space that is in the opening and is sandwiched between the cover 19 and the enzyme reagent layer 17/the insulating layer 14 serves as a sample supply portion 21 having a capillary structure. Moreover, the through hole 20 serves as an air hole for drawing a sample in by capillary action.

The size of the biosensor 1 is not particularly limited and can be set as appropriate depending on the amount of a sample to be supplied or the like. For example, the total length can be 5 to 50 mm, the total width can be 1 to 50 mm, the maximum thickness can be 500 to 2000 $\mu$m, and the minimum thickness can be 50 to 500 $\mu$m. It should be noted that "length" of each portion refers to the length in the longitudinal direction of the biosensor, and "width" refers to the length in the width direction of the biosensor (the same applies hereinafter).

The size of the substrate 11 is, for example, 5 to 50 mm for the length, 1 to 50 mm for the width, and 10 to 1000 $\mu$m for the thickness. The size of the insulating layer 14 is, for example, 5 to 50 mm for the length, 1 to 50 mm for the width, and 10 to 200 $\mu$m for the thickness. The size of the detecting portion 15 is, for example, 0.1 to 10 mm for the length and 0.1 to 10 mm for the width. The size of the inorganic gel layer 16 is, for example, 0.1 to 10 mm for the length, 0.1 to 10 mm for the width, and 0.001 to 500 $\mu$m for the thickness. The size of the enzyme reagent layer 17 is, for example, 0.1 to 10 mm for the length, 0.1 to 10 mm for the width, and 0.001 to 500 $\mu$m for the thickness. The size of the spacer is, for example, 1 to 50 mm for the length, 1 to 50 mm for the width, and 10 to 1000 $\mu$m for the thickness, and the size of the opening provided therein is, for example, 0.1 to 10 mm for the length and 0.01 to 10 mm for the width. The size of the cover 19 is, for example, 5 to 50 mm for the length, 1 to 50 mm for the width, and 10 to 1000 $\mu$m for the thickness, and the size of the through hole provided therein is, for example, 0.1 to 10 mm for the diameter.

The content of the layered inorganic compound in the inorganic gel layer 16 can be determined as appropriate depending on the type or the amount of a sample to be supplied, the area of the detecting portion 15, or the like. For example, the content of the layered inorganic compound is in the range from 0.003 to 30 mg, preferably from 0.1 to 10 mg, and more preferably 0.3 to 3 mg per $cm^2$ of the detecting portion 15. More specifically, in the case where the layered inorganic compound is smectite, the content thereof is, for example, in the range from 0.003 to 30 mg, preferably from 0.1 to 10 mg, and more preferably 0.3 to 3 mg per $cm^2$ of the detecting portion 15. When the content of the layered inorganic compound per $cm^2$ of the detecting portion 15 is 0.003 mg or more, a sufficient oxygen-blocking effect can be obtained, for example. On the other hand, when the content of the layered inorganic compound per $cm^2$ of the detecting portion 15 is 30 mg or less, it is possible to further improve the oxygen-blocking effect, reproducibility, and reactivity. Note here that the amount of the layered inorganic compound per unit area of the detecting portion 15 is pertinent to the thickness of the inorganic gel layer 16, and it is preferable that the thickness of the inorganic gel layer 16 is in the range from 0.001 to 500 $\mu$m as described above.

The content of a surfactant (i.e., an ampholytic surfactant) in the inorganic gel layer 16 can be determined as appropriate, for example, depending on the amount of the layered inorganic compound. The content of a surfactant is, for example, in the range from 0.1 mmol to 100 mmol, preferably from 0.5 mmol to 10 mmol, and more preferably from 0.5 mmol to 1 mmol with respect to 300 mg of the layered inorganic compound.

The content of a mediator in the inorganic gel layer 16 can be determined as appropriate, for example, depending on the type of a sample to be measured, the type of an analyte, the amount of an oxidoreductase in the enzyme reagent layer, which will be described later, or the like. However, it is preferable that the content of a mediator is, for example, in the range from 10 mmol to 100 mol, more preferably from 10 mmol to 50 mmol, and particularly preferably from 15 mmol to 20 mmol per $cm^2$ of the detecting portion 15.

The inorganic gel layer 16 further may contain a buffer. The content of the buffer in the inorganic gel layer 16 can be determined as appropriate, for example, depending on the amount of the layered inorganic compound. Preferably, the content of the buffer is, for example, in the range from 0.1 mmol to 100 mmol, more preferably from 1 mmol to 50 mmol, and particularly preferably from 10 mmol to 20 mmol with respect to 0.3 g of the layered inorganic compound.

The content of an oxidoreductase in the enzyme reagent layer 17 is not particularly limited and can be determined as appropriate depending on the type or the amount of a sample, the type or the amount of an analyte, or the like. Specifically, the content of an oxidoreductase is, for example, in the range from 0.1 U to 100 KU, preferably from 1 U to 10 KU, and more preferably from 1 U to 100 U per $cm^2$ of the detecting portion 15.

Furthermore, the amount of the mediator contained in the inorganic gel layer 16 relative to the amount of the enzyme contained in the enzyme reagent layer 17 is as follows, for example: 0.01 to 1 M, preferably 0.01 to 0.5 M, and more preferably 50 mM to 200 mM of the mediator is present with respect to 1000 U of the enzyme.

Such a biosensor can be produced, for example, in the following manner.

First, the substrate 11 on which the electrode system is to be formed is provided. The substrate 11 preferably is formed of an electrically insulating material, such as plastics, glass, paper, ceramics, and rubbers. Examples of the plastics include polyethylene terephthalate (PET), polystyrene (PS), polymethacrylate (PMMA), polypropylene (PP), acrylic resin, and glass epoxy.

Next, as shown in FIG. 1A, the electrode system including the working electrode 12 having the lead portion 12a and the counter electrodes 13 having the lead portion 13a is formed on the substrate 11. Note here that the shapes of the electrodes are by no means limited to those shown in FIG. 1A. As the electrodes, carbon electrodes, gold electrodes, palladium electrodes, platinum electrodes, or the like are preferable, and the electrodes can be formed by a known method such as screen printing, coating, or an evaporation method, depending on the type thereof.

The carbon electrodes can be formed by, for example, means for screen-printing or coating carbon ink on the substrate 11.

The gold electrodes can be formed by, for example, an evaporation method, plating, sputtering, a gold foil attachment method, or the like. The evaporation method is a method performed in the following manner, for example. First, gold is deposited on a plastic sheet such as PET by, for example, ion plating at a vacuum degree of $1.33 \times 10^{-4}$ Pa, an input power of 300 W, and a rate of $5 \times 10^{-1}$ nm/sec for 2 minutes. Then, the gold foil layer deposited on the sheet is cut in the form of a thin line with a kisscut device. Thus, the cut in the form of a thin line serves as the insulating portion, so that the electrode system including the working electrode and the counter electrode can be formed.

Next, as shown in FIG. 1B, the insulating layer 14 is formed on the substrate 11 on which the electrodes 12 and 13 have been formed. This insulating layer is formed on the substrate 11, except on the lead portions 12a and 13a (on the left in FIG. 1B) and the detecting portion 15 on which an inorganic gel layer and the like that will be described later are to be formed.

The insulating layer 14 can be formed by, for example, printing an insulating paste prepared by dissolving an insulating resin in a solvent on the substrate 11, and subjecting it to a heat treatment or an ultraviolet treatment.

Examples of the insulating resin include polyester, butyral resin, and phenolic resin. Examples of the solvent include carbitol acetate and mixed solvents based on dibasic acid esters (DBE solvents). The concentration of the insulating resin in the paste preferably is in the range from 65 to 95 wt%, more preferably from 75 to 90 wt%, and particularly preferably from 80 to 85 wt%, for example.

Conditions for the heat treatment can be determined as appropriate depending on the type of the insulating resin used.

Note here that, in addition to the printing as described above, the insulating layer 14 can be formed by coating, film attachment, etching, or other methods.

Next, as shown in FIG. 1C, in the detecting portion 15 on which the insulating layer 14 is not formed, the inorganic gel layer 16 is formed on the substrate 11 and the electrodes 12 and 13. The inorganic gel layer 16 can be formed by, for example, preparing a layered inorganic compound dispersion that contains a mediator and a surfactant, dispensing the dispersion into the detecting portion 15, and then drying it. Note here that it is not always necessary that the inorganic gel layer 16 is in the form of gel at all times. The inorganic gel layer 16 may be in a dried state achieved by the above-described drying treatment before use, and preferably is turned into gel when impregnated with a liquid sample or the like.

Examples of the solvent used for preparing the dispersion include water, buffer solutions, alcohols, N,N-dimethylformamide (DMF), and dimethylsulfoxide (DMSO). Among these, ultrapure water is preferable.

The concentration of the layered inorganic compound in the dispersion is, for example, in the range from 0.1 to 100 mg/ml, preferably from 1 to 100 mg/ml, and more preferably from 10 to 30 mg/ml. Furthermore, the concentration of the surfactant in the dispersion is, for example, in the range from 1 to 200 mM, preferably from 1 to 100 mM, and more preferably from 1 to 10 mM.

The layered inorganic compound and the surfactant are contained in the dispersion so that, for example, 0.1 mmol to 100 mmol, preferably 0.5 mmol to 10 mmol, more preferably 0.5 mmol to 1 mmol of the surfactant is present with respect to 300 mg of the layered inorganic compound.

Furthermore, the concentration of the mediator in the dispersion is, for example, in the range from 1 to 1000 mM, preferably from 100 to 800 mM, more preferably from 200 to 500 mM, and particularly preferably around 300 mM.

Preferably, the dispersion further contains a buffer such as an amine buffer as described above. In this case, the concentration of the buffer is, for example, in the range from 1 to 1000 mM, preferably from 10 to 500 mM, and more preferably from 50 to 200 mM. Furthermore, the amount of the buffer to be contained in the dispersion relative to the amount of the layered inorganic compound is as follows, for example: the amount of the buffer preferably is in the range from 0.1 mmol to 100 mmol, more preferably from 1 mmol to 50 mmol, and particularly preferably from 10 mmol to 20 mmol with respect to 0.3 g of the layered inorganic compound.

The amounts of the surfactant and the buffer to be contained in the dispersion can be determined, for example, based on the amount of the layered inorganic compound as described above. Accordingly, the ratio (molar ratio A: B) of the amount of the surfactant (A) to the amount of the buffer (B) to be contained in the dispersion is, for example, in the range from 1 : 1 to 1 : 250, preferably from 1 : 10 to 1 : 100, and more preferably from 1 : 25 to 1 : 50, though there is no particular limitation regarding the ratio.

The order of adding the respective components such as the mediator, the layered inorganic compound, and the surfactant to the solvent is not particularly limited, but they preferably are added in the following order, for example. First, the layered inorganic compound is added to the solvent. After the mixture is stirred sufficiently, the surfactant is added. Thereafter, the buffer is added, and finally, the mediator is dissolved in the mixture. When the components are added in this order, it is possible to form a still more uniform inorganic gel layer that can prevent the contact with oxygen still more effectively, although the mechanism is unknown. The buffer and the surfactant may be added simultaneously, but it is particularly preferable to add the buffer after adding the surfactant.

Though there is no particular limitation regarding the method for preparing the dispersion, it is preferable to adjust the pH of the dispersion or to select the type of the buffer to be used, for example, depending on the type of the mediator to be used or the like. Specific examples will be given below with regard to various types of mediators.

First, an example where a mediator preferably used in the vicinity of neutral pH is used will be described. In general, a layered inorganic compound (inorganic gel) like smectite becomes transparent when dissolved in a solvent such as water, and the dispersion thereof is strongly alkaline (with a pH of around 10). On the other hand, it is preferable that a mediator, an enzyme, etc. preferably used in the vicinity of neutral pH are added to the dispersion under stable pH conditions, i.e., in the vicinity of neutral pH. However, the inorganic gel may become cloudy or may be precipitated out of the dispersion in the vicinity of neutral pH. In this case, if the above-described amine buffer is added to the dispersion as a buffer, the pH of the dispersion can be adjusted to be in the vicinity of neutral pH, and besides, although the mechanism is unknown, the mediator can be added without causing precipitation even though the pH of the dispersion is in the vicinity of neutral pH. As a result, an inorganic gel layer that can prevent the reoxidization of the mediator sufficiently as described above can be formed reliably. It should be noted that the inventors of the present invention also found for the first time that, by adding an amine buffer after adding a surfactant like an ampholytic surfactant, it is possible to prevent sufficiently the prepared dispersion from becoming cloudy and precipitation from being caused in the dispersion. More specifically, it is preferable to add a surfactant and then an amine buffer to the dispersion of the layered inorganic compound. The pH of the dispersion after the amine buffer has been added thereto is, for example, in the range from 9 to 5, preferably from 8 to 6, more preferably from 7.5 to 7.

Examples of the mediator preferably used in the vicinity of neutral pH include potassium ferricyanide, cytochrome c, PQQ, $NAD^+$, $NADP^+$, and copper complexes.

Furthermore, when a mediator preferably used in the vicinity of acidic pH is used, a dispersion preferably is prepared in the following manner. As already described above, a layered inorganic compound (inorganic gel) generally becomes transparent when dissolved in a solvent, and the dispersion thereof is strongly alkaline. In this case, in order to add a mediator preferably used in the vicinity of acidic pH, it is preferable that, for example, the pH of the dispersion is adjusted so as to be acidic by adding an acid such as HCl to the dispersion, the surfactant is then added to the dispersion, and thereafter, the pH of the dispersion is adjusted again by adding the buffer having a carboxyl group as described above, and then the mediator is added to the dispersion.

The above-described method is preferable for the following reason. When a dispersion of a layered inorganic compound such as smectite is made strongly acidic (e.g. a pH of around 2) by adding an acid such as HCl thereto, the dispersion becomes cloudy due to the aggregation. However, the inventors of the present invention found that further stirring (e.g., for about 24 hours) allows the dispersion, which is acidic, to become transparent again. However, the dispersion in this state does not contain a buffer serving as a binder as described above, so that the effect produced by the buffer cannot be obtained. Moreover, the strongly acidic dispersion with a pH of around 2 is inconvenient taking the use of an enzyme into account, because there is a possibility that the enzyme might be deactivated. Thus, the inventors of the present invention conducted further studies and found that, by adding a surfactant like an ampholytic surfactant to the strongly acidic dispersion that has been made transparent and then adding a buffer containing a carboxyl group to adjust the pH of the dispersion within a range (e.g., around 4.5) causing no interference with the respective components such as the mediator and the enzyme, precipitation can be prevented sufficiently and the mediator and the enzyme can be used in a sufficiently stable state.

It is preferable that the dispersion initially is strongly acidic with a pH of 1 to 3, more preferably 1.5 to 2. The acid to be used is not particularly limited, but is, for example, hydrochloric acid, phosphoric acid, acetic acid, or the like. There is no particular limitation regarding the conditions for stirring the dispersion under strongly acidic conditions, but the stirring period is, for example, 12 to 72 hours, preferably 18 to 48 hours, and particularly preferably 24 to 30 hours. It is preferable that the pH of the dispersion is set to 3 to 6, more preferably 4 to 5, and particularly preferably 4.5 to 4.8 by adding a buffer having a carboxyl group thereto.

Examples of mediator preferably used in the vicinity of acidic pH include ruthenium complexes, osmium complexes, ferrocene, phenazine methosulfate, indophenol, and methylene blue.

After the layered inorganic compound, the surfactant, and the buffer have been added to the solvent, it is preferable that the mixture is allowed to stand still for a certain period, preferably at least 24 hours, more preferably at least 3 days, for example.

The amount of the dispersion to be poured into the detecting portion 15 can be determined as appropriate, for example, depending on the size of the detecting portion 15, the content of the layered inorganic compound or the like in the dispersion, the type of the layered inorganic compound, or the like. More specifically, it is preferable to pour the dispersion so that the amount of the layered inorganic compound per unit area ($cm^2$) of the detecting portion 15 is, for example, in the range from 0.003 to 30 mg, more preferably from 0.1 to 10 mg, and particularly preferably from 0.3 to 3 mg. Accordingly, in the case where the concentration of the layered inorganic compound in the dispersion is 0.3 wt%, the amount of the dispersion to be poured is, for example, in the range from 0.001 to 10 ml, more preferably from 0.03 to 3.3 ml, and particularly preferably 0.1 to 1 ml per unit area ($cm^2$) of the detecting portion.

There is no particular limitation regarding the method of pouring the dispersion into the detecting portion 15, but the dispersion can be poured into detecting portion 15 using an automatically driven dispenser or the like, for example.

There is no particular limitation regarding the means for drying the poured dispersion. For example, natural drying, air drying, drying under reduced pressure, lyophilization under reduced pressure, or the like can be used. These methods can be used in combination. In the drying treatment, the treatment temperature preferably is in the range from 10°C to 60°C, more preferably from 25°C to 50°C, and particularly preferably from 30°C to 40°C. Furthermore, the relative humidity preferably is in the range from 5% RH to 40% RH, more preferably from 10% RH to 20% RH, and particularly preferably from 10% RH to 15% RH. The treatment time can be determined as appropriate, for example, depending on the means for drying, but preferably is in the range from 1 to 60 minutes, more preferably from 5 to 30 minutes, and particularly preferably from 5 to 10 minutes.

Furthermore, as shown in FIG. 1D, the enzyme reagent layer 17 is formed on the inorganic gel layer 16. This enzyme reagent layer 17 can be formed by preparing an enzyme solution containing an oxidoreductase, pouring the enzyme solution onto the inorganic gel layer 16, and then drying it. As the oxidoreductase, those described above can be used, for example.

The enzyme solution can be prepared by dissolving an enzyme in a solvent sufficiently. There is no particular limitation regarding the solvent. Examples of the solvent include water, buffer solutions, and organic solvents such as ethanol, methanol, butanol, dimethylsulfoxide (DMSO), and tetrahydrofuran. The buffer solution can be a phosphate buffer solution, a citrate buffer solution, an acetate buffer solution, a Tris-HCl buffer solution, or a Good's buffer solution, for example. The pH of the buffer solution can be determined as appropriate depending on the type of the enzyme, but preferably is, for example, in the range from 5 to 10, more preferably from 6 to 9, and particularly preferably from 7 to 8. Examples of the water include purified water, distilled water, and ultrapure water. Among these, ultrapure water is preferable, because a highly accurate biosensor that hardly contains impurities can be produced.

The concentration of a reagent in the enzyme solution is not particularly limited, but preferably is, for example, in the range from 1 to 10 KU/ml, more preferably from 3 to 6 KU/ml.

It is preferable that the enzyme solution further contains a surfactant, particularly preferably an ampholytic surfactant. As the ampholytic surfactant, those described above can be used, for example.

In addition to the oxidoreductase, the following components also may be contained in this enzyme solution, for example: saccharides that do not serve as substrates for the oxidoreductase, amino acids and derivatives thereof, amine compounds such as imidazole, betaines, and the like. Examples of the saccharides include sucrose, raffinose, lactitol, ribitol, and arabitol. Among these, the saccharides can be added for the purpose of, for example, improving the stability of the enzyme, the amino acids and derivatives thereof and betaines can be added for the purpose of, for example, preventing the reagent from hardening by being dried, and imidazole can be added for the purpose of, for example, stabilizing the mediator.

The amount of the enzyme solution to be poured can be determined as appropriate depending on the size of the enzyme reagent layer 17 to be formed, the concentration of the reagent, the amount of the sample, the type of the analyte or the like.

There is no particular limitation regarding the means for drying the poured enzyme solution. For example, natural drying, air drying, drying under reduced pressure, lyophilization under reduced pressure or the like can be used. These methods can be used in combination. As the drying conditions, for example, the temperature is in the range from 10°C to 60°C, the relative humidity is in the range from 5% RH to 40% RH, and the time is in the range from 1 to 60 minutes. In the case where an enzyme is used as the reagent, the temperature may be set as appropriate depending on the type of the enzyme so as not to deactivate the enzyme.

Next, as shown in FIG. 1E, the spacer 18 is disposed on the insulating layer 14. As shown in FIG. 1E, the spacer 18 has an opening at the portion corresponding to the enzyme reagent layer 17.

The spacer 18 can be made of, for example, a resin film or tape. If it is a double-faced tape, not only the insulating layer 14 but also the cover 19 that will be described later can be attached easily. In addition to that, the spacer can be formed by resist printing or other means, for example.

Next, as shown in FIG. 1F, the cover 19 is disposed on the spacer 17. There is no particular limitation regarding the material of the cover 19. For example, various plastics can be used, and preferably transparent resin such as PET can be used.

It is preferable that the thus-produced biosensor 1 is stored air-tightly together with a desiccant such as molecular sieves, silica gel, or calcium oxide in order not to be affected by humidity when it is stored for a long time.

The biosensor 1 can be used in combination with measuring equipment provided with, for example, means for applying a predetermined voltage for a certain time, means for measuring an electrical signal transmitted from the biosensor, means for calculating the electrical signal into the concentration of the analyte, and other means.

The use of the biosensor 1 will be described by taking an example in which a sample is whole blood, the analyte is glucose, the oxidoreductase is GDH, and the mediator is potassium ferricyanide.

First, the whole blood sample is brought into contact with one end of the opening 21 of the biosensor 1. This opening 21 has a capillary structure as described above, and the air hole 20 is provided in the cover 19 at the portion corresponding to the other end thereof, so that the sample is drawn in by capillary action. The drawn sample permeates the enzyme reagent layer 17 provided on the detecting portion 15. The sample dissolves GDH contained in the enzyme reagent layer 17 and reaches the surface of the inorganic gel layer 16 provided below the enzyme reagent layer 17. Then, reactions are caused by the glucose and the GDH contained in the sample that has reached the surface of the inorganic gel layer 16 and potassium ferricyanide contained in the inorganic gel layer 16. More specifically, the glucose as an analyte is oxidized by the GDH, and the potassium ferricyanide is reduced by electrons that have been moved by this oxidation reaction, so that potassium ferrocyanide (ferrocyanide ions) is produced.

It is to be noted that, although the sample passes through the enzyme reagent layer 17 to reach the inorganic gel layer 16, it never passes through the inorganic gel layer 16 to reach the surfaces of the electrodes. It is considered that this prevents the reduced potassium ferrocyanide from being reoxidized by dissolved oxygen present in the sample, thereby suppressing the deterioration of the measurement accuracy. Note here that the fact that no moisture reaches the surfaces of the electrodes has been attested by the observation with an electron microscope. Moreover, impurities such as erythrocytes contained in the sample also cannot pass through the layered inorganic compound, so that they cannot pass through the inorganic gel layer 16 and are prevented from being adsorbed at the surfaces of the electrodes 12 and 13.

Then, electrons are transferred between the potassium ferrocyanide reduced in the inorganic gel layer 16 and the electrodes located below the inorganic gel layer 16, whereby the glucose concentration can be measured. More specifically, the measurement can be achieved in the following manner.

After a predetermined time has passed from the supply of the whole blood sample, a voltage is applied between the counter electrode 13 and the working electrode 12 by the means for applying a voltage, so that the reduced potassium ferrocyanide (ferrocyanide ions) that is in contact with the electrodes is oxidized electrochemically into potassium ferricyanide, and the oxidation current at that time is detected by, for example, the means for measuring an electrical signal via the lead portion 12a of the working electrode 12. The value of the oxidation current is proportional to the glucose concentration in the sample, so that the glucose concentration in the sample can be obtained by using the oxidation current to calculate the glucose concentration with the calculating means.

According to such a biosensor, the reoxidation of the reduced mediator due to the influence of dissolved oxygen or the like can be prevented as described above, so that the accuracy and reproducibility of the measurement can be improved.

In this embodiment, an example where a biosensor of the present invention is used for measuring glucose has been shown, but the present invention is not limited thereto. For example, the biosensor can be used for measuring various analytes by determining the reagent as appropriate depending on the type of the analyte. More specifically, for example, lactate oxidase can be used as the reagent to provide a biosensor for measuring lactic acid, alcohol oxidase can be used as the reagent to provide a biosensor for measuring alcohol, and cholesterol oxidase or the like can be used as the reagent to provide a biosensor for measuring cholesterol. Furthermore, pyranose oxidase or glucose oxidase also can be used as the reagent to provide a biosensor for measuring glucose, for example.

Moreover, instead of laminating the enzyme reagent layer on the inorganic gel layer, a single layer that serves as an enzyme reagent layer and an inorganic gel layer may be formed in the detecting portion 15 by further adding an oxidoreductase to the above-described dispersion containing the layered inorganic compound etc. In this case, the amount of the oxidoreductase to be contained in the dispersion is not particularly limited, and can be determined with reference to the description with regard to the amount of the oxidoreductase in the above, for example.

Example 1

A glucose sensor having the same structure as that shown in FIG. 1F was produced in the following manner.

First, a substrate made of PET (with a length of 50 mm, a width of 6 mm, and a thickness of 250 $\mu$m) was provided as an insulating substrate 11 of a glucose sensor, and a carbon electrode system including a working electrode 12 and a counter electrode 13, each of which had a lead portion, was formed on one surface of the substrate by screen printing.

Next, an insulating layer 14 was formed on the electrodes in the following manner. First, polyester as an insulating resin was dissolved in carbitol acetate as a solvent so that its concentration became 75 wt% to prepare insulating paste, and the thus-obtained insulating paste was screen-printed on the electrodes. The printing was performed under the conditions of 300 mesh screen and a squeegee pressure of 40 kg, and the amount of the insulating paste used for the printing was 0.002 ml per cm$^2$ of the electrode area. The screen printing was not performed on a detecting portion 15 and the lead portions 12a and 13a. Then, a heat treatment was performed at a temperature of 90°C for 60 minutes. Thus, the insulating layer 14 was formed.

Then, on the detecting portion 15 on which the insulating layer 14 was not formed, an inorganic gel layer 16 was formed in the following manner. First, 0.6 g of a product named "LUCENTITE SWN" (CO-OP CHEMICAL Co. Ltd.) as a synthesized smectite was suspended in 100 ml of purified water and stirred for about 8 to 24 hours. The thus-obtained synthesized smectite suspension had a pH of about 10. To 10 ml of this synthesized smectite suspension, 0.1 ml of a 10% (w/v) aqueous solution of CHAPS Dojindo Laboratories), 5.0 ml of a 1.0M ACES buffer solution (pH 7.4, Dojindo Laboratories), and 4.0 ml of purified water were added in this order, and then 1.0 g of $[Ru(NH_3)_6]Cl_3$ (Aldrich) further was added as a mediator. The resultant mixture was used as a solution for forming an inorganic gel layer (hereinafter referred to as an "inorganic gel layer-forming solution") (pH 7.5). The final concentrations of the respective constituents in this inorganic gel layer-forming solution are shown below.

| | |
|---|---|
| LUCENTITE SWN | 0.3% (w/v) |
| CHAPS | 0.3% (w/v) |
| ACES buffer solution (pH 7.5) | 100 mM |
| $[Ru(NH_3)_6]Cl_3$ | 5.0% (w/v) |

1.0 $\mu$l of this inorganic gel layer-forming solution was dispensed into the detecting portion 15. The surface area of the detecting portion 15 was about 0.1 cm$^2$, and the surface area of the electrodes 12 and 13 in the detecting portion 15 was about 0.12 cm$^2$. The inorganic gel layer-forming solution was dried for 10 minutes at 30°C and a relative humidity of 10% RH. Thus, the inorganic gel layer 16 was formed.

On the inorganic gel layer 16, an enzyme reagent layer 17 further was formed. The enzyme reagent layer 17 was formed by dispensing 1.0 $\mu$l of a 5000 U/ml GDH aqueous solution onto the inorganic gel layer 16 formed in the detecting portion 15 and then drying it for 10 minutes at 30°C and a relative humidity of 10% RH.

Finally, a spacer 18 having an opening was disposed on the insulating layer 14, and a cover 19 having a through hole 20 serving as an air hole was disposed on the spacer 18. Thus, a biosensor 1 was produced. A space that was in the opening of the spacer 18 and sandwiched between the cover 19 and the insulating layer 14 had a capillary structure. Thus, this space was used as a sample supply portion 21.

Furthermore, as a glucose sensor according to Comparative Example 1, a glucose sensor was produced in the same manner as in Example 1, except that the inorganic gel layer-forming solution was prepared using purified water instead of the synthesized smectite suspension.

Example 2

In Example 2, using the glucose sensor produced in Example 1, the change in response current over time was measured with regard to samples having various glucose concentrations.

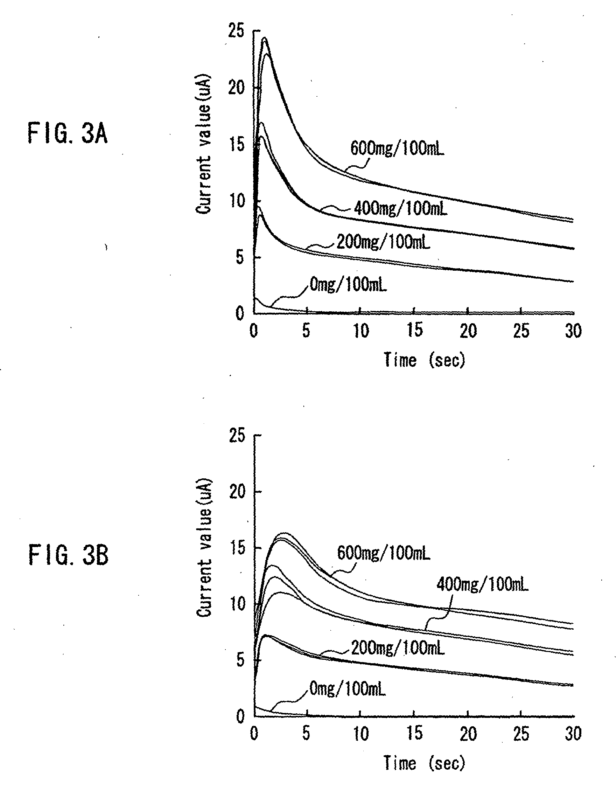

Human whole blood was used to prepare liquid samples. First, the whole blood collected was left at 37°C for about 1 day, and the glucose concentration thereof was adjusted to be 0 mg/100 ml. Then, to this whole blood, glucose was added so as to prepare samples having various glucose concentrations (about 200, 400, and 600 mg/100 ml). The whole blood to which no glucose was added was used as a sample having a glucose concentration of 0 mg/100 ml. In Example 2, each of the samples was dropped on the sample supply portion 21 after the start of voltage application (200 mV) to the glucose sensor 1, and the time course of the change in response current was started to be measured after a lapse of 5 seconds from the dropping of the sample. Furthermore, in Comparative Example 2, the same measurement was carried out using the glucose sensor according to Comparative Example 1. In both Example 2 and Comparative Example 2, the measurement was carried out three times in total (n = 3). The results are shown in FIGs. 3A and 3B. FIG. 3 is a graph showing the change in measured current over time when each of the samples was measured using the respective glucose sensors, wherein FIG. 3A shows the results obtained in Example 2 and FIG. 3B shows the results obtained in Comparative Example 2.

As shown in FIG. 3A, the peak response current in the measurement using the glucose sensor of Example 1 appeared earlier and also was greater than that in the measurement using the glucose sensor of Comparative Example 1 shown in FIG. 3B. The reason why the greater peak current value was obtained is considered to be as follows. In the glucose sensor of Example 1, since oxygen was blocked by the inorganic gel layer, the mediator having been reduced by the reaction between the glucose and GDH could be oxidized electrochemically without being reoxidized by oxygen. Thus, the oxidation current obtained through this electrochemical oxidation could be measured. On the other hand, the reason why the peak current appeared earlier is considered to be as follows. In the glucose sensor of Example 1, in the inorganic gel layer formed on the electrodes, $[Ru(NH_3)_6]Cl_3$ as the mediator was intercalated and immobilized firmly between sheets of smectite. This brought about the state where the mediator was held loosely on the surface of the electrodes via smectite. Accordingly, the concentration of the mediator was high in the vicinity of the electrodes, resulting in increased reaction velocity.

Example 3

In Example 3, using a glucose sensor of the present invention, the reproducibility of the response current value obtained after a lapse of a certain period from the dropping of a sample was examined.

Glucose was added to human whole blood so as to prepare samples with glucose concentrations of 0, 103, 415, 616, and 824 mg/100 ml.

As a glucose sensor according to Example 3, a glucose sensor was produced in the same manner as in Example 1, except that the concentration of smectite (LUCENTITE SWN) in the inorganic gel layer-forming solution was set to 0.24% (w/v). Each of the samples was dropped on the glucose sensor after the start of voltage application (200 mV) between the electrodes of the glucose sensor. Then, the response current was measured after a lapse of 5 seconds from the dropping of the sample. Using the same glucose sensor, the above-described measurement was carried out 10 times in total (n = 10) with regard to each sample. Furthermore, in Comparative Example 3, the same measurement as in Example 3 was carried out using the glucose sensor of Comparative Example 1 (n = 10).

Based on the response current values (n = 10) obtained in Example 3 and Comparative Example 3, a CV value representing the reproducibility of the measurement was determined. The results are shown in Table 2 below.

[Table 2]

|  | Concentration of glucose in sample | | | |
|  | 103mg/100ml | 415mg/100ml | 616mg/100ml | 824mg/100ml |
| --- | --- | --- | --- | --- |
| Ex. 3 | 3.02% | 2.34% | 2.90% | 2.33% |
| Comp. Ex. 3 | 3.84% | 3.31% | 3.35% | 7.30% |

As can be seen from the result shown in Table 2, in Comparative Example 3, when the glucose concentration in the sample was increased to 824 mg/100 ml, the CV value changed drastically to 7.30. In contrast, in Example 3, the glucose sensor exhibited a stable CV value that varied within the small range from 2.34 to 3.02. This demonstrates that the glucose sensor of Example 3 could carry out the measurement with high reproducibility regardless of the increase in glucose concentration. From these results, it can be said that the glucose sensor according to Example 3 can perform measurement with higher reproducibility than the biosensor of Comparative Example 1 containing no smectite.

Example 4

In Example 4, with regard to the glucose sensor produced in Example 3, the influence exerted upon the glucose sensor when it is exposed to a certain humidity and a certain temperature was examined.

The glucose sensor was left in a room maintained at a relative humidity of 80% RH and a temperature of 40°C for 17 hours, after which voltage application (200 mV) between the electrodes was started. Then, each of human whole blood samples prepared by adding glucose to human whole blood so that the glucose concentrations became 0 and 600 mg/100 ml was dropped on the glucose sensor, and the response current was measured after a lapse of 5 seconds from the dropping of the sample. The above measurement is regarded as Example 4. Furthermore, as Comparative Example 4, the same measurement was carried using the glucose sensor of Comparative Example 1. Still further, as control experiments for Example 4 and Comparative Example 4, the measurement of the response current was carried out with regard to the same sample at ordinary room temperate and humidity (about 25°C and about 60% RH), using the glucose sensors used in Example 4 and Comparative Example 4. The sensitivities (%) of the glucose sensors used in Example 4 and Comparative Example 4 respectively were determined by indicating the response current values obtained in Example 4 and Comparative Example 4 as relative values with respect to those obtained in the corresponding control exterminates as 100%. The results are shown in Table 3 below.

[Table 3]

|  | Sensitivity (%) |
| --- | --- |
| Example 4 | 74.2% |
| Comparative Example 4 | 27.6% |

As shown in Table 3, even after being exposed to high humidity conditions, the deterioration of the sensitivity in the glucose sensor of Example 3 was smaller than that in the glucose sensor of Comparative Example 1. That is, the glucose sensor of Example 3 is resistant to humidity, because the inorganic gel layer can block, for example, moisture in the air or dissolved oxygen in the sample so that the mediator is prevented from being brought into contact with oxygen as described above.

Example 5

In Example 5, with regard to a glucose sensor of the present invention, the influence of dissolved oxygen in a sample was examined.

To human whole blood, glucose was added so as to prepare samples with a glucose concentration of 111 mg/ 100 ml. The amounts of dissolved oxygen in these samples were adjusted so as to be in the vicinity of 25.1 mmHg (unadjusted), 92.0 mmHg, and 171.6 mmHg, respectively. The adjustment to the higher dissolved oxygen concentrations (92.0 mmHg and 171.6 mmHg) was achieved by mixing the sample having the unadjusted dissolved oxygen concentration (25.1 mmHg) with oxygen in a test tube.

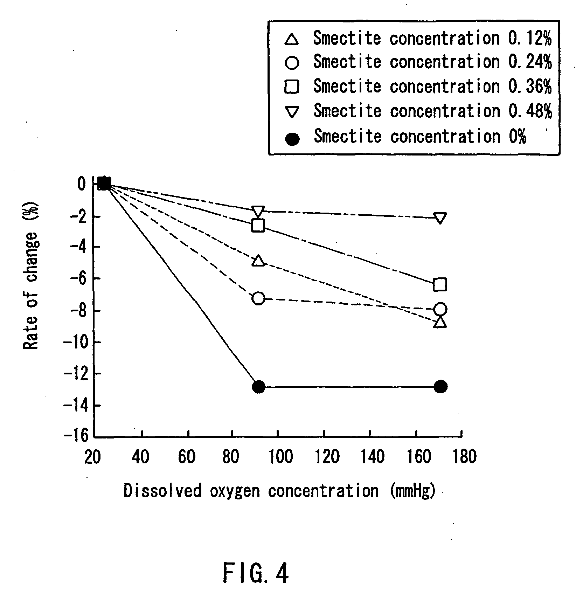

As glucose sensors according to Example 5, four types of glucose sensors were produced in the same manner as in Example 1, except that the concentrations of smectite (LUCENTITE SWN) in the inorganic gel layer-forming solution were set to 0.12%, 0.24%, 0.36%, and 0.48% (w/v). Each of the samples was dropped on the respective glucose sensors after the start of voltage application (200 mV) between the electrodes of each glucose sensor. Then, the response current was measured after a lapse of 5 seconds from the dropping of the sample. With regard to each of these glucose sensors, the rate of change (%) in the response current value obtained in the measurement of each of the samples with the adjusted dissolved oxygen concentrations (92.0 mmHg and 171.6 mmHg) relative to the response current value obtained in the measurement of the sample with the unadjusted dissolved oxygen concentration (25.1 mmHg) was determined using the following formula (10). Furthermore, as Comparative Example 5, the measurement and the determination of the rate of change (%) were carried out in the same manner as in Example 5 with regard to the glucose sensor of Comparative Example 1. The results are shown in FIG. 4. FIG. 4 is a graph showing the relationship between the concentration of dissolved oxygen in the sample and the rate of change (%). In FIG. 4, $\Delta$, o, $\square$, and $\nabla$ indicate the results obtained in Example 5, and • indicates the results obtained in Comparative Example 5. Note here that as the absolute value of the rate of change (%) is greater, the change in response current is more significant.

$$\text{Rate of change (\%)} = [(A/B) - 1] \times 100 \qquad \dots (10)$$

A: response current obtained in the measurement of a sample with adjusted dissolved oxygen concentration
B: response current obtained in the measurement of a sample with unadjusted dissolved oxygen concentration

As shown in FIG. 4, the glucose sensors of Example 5 showed smaller absolute values of the rate of change (%) than the glucose sensor of Comparative Example 5 even if the concentration of dissolved oxygen in the sample was increased. This demonstrate that the deterioration of the sensitivity due to dissolved oxygen is small in the glucose sensors of Example 5 containing smectite and thus these sensors are less susceptible to the influence of dissolved oxygen present in a solution such as a sample.

Example 6

In Example 6, the influence of dissolved oxygen in a sample was examined with regard to a glucose sensor of the present invention in which GOD was used as an oxidoreductase.

A glucose sensor was produced in the same manner as in Example 1, except that: a dispersion prepared so as to contain the respective constituents at the final concentrations shown below was used as the inorganic gel layer-forming solution; a 1200 U/ml GOD solution (Amano Enzyme Inc.) was used as the enzyme solution instead of the GDH aqueous solution; potassium ferricyanide (Wako Pure Chemical Industries, Ltd.) was used instead of $[Ru(NH_3)_6]Cl_3$; and a Tris-HCl buffer solution (pH7.4: Dojindo Laboratories) was used instead of the ACES buffer solution.

| | |
|---|---|
| LUCENTITE SWN | 0.3% (w/v) |
| CHAPS | 0.1% (w/v) |
| Tris-Hcl buffer solution | 100 mM |
| Potassium ferricyanide | 3.0% (w/v) |

Furthermore, as a glucose sensor according to Comparative Example 6, a glucose sensor was produced in the same manner as in Example 1, except that, instead of forming the inorganic gel layer and the enzyme reagent layer, 1 $\mu$l of a solution containing 1200 U/ml of GOD and 3.0% (w/v) of potassium ferricyanide was poured into the detecting portion and dried.

To human whole blood, glucose was added so as to prepare samples with a glucose concentration of 600 mg/ 100 ml. The amounts of dissolved oxygen in these samples were adjusted so as to be 48.9 mmHg (unadjusted), 106.4 mmHg, and 180.4 mmHg, respectively. The adjustment to higher dissolved oxygen concentrations was achieved by mixing the sample having the unadjusted dissolved oxygen concentration with oxygen in a test tube, as in Example 5.

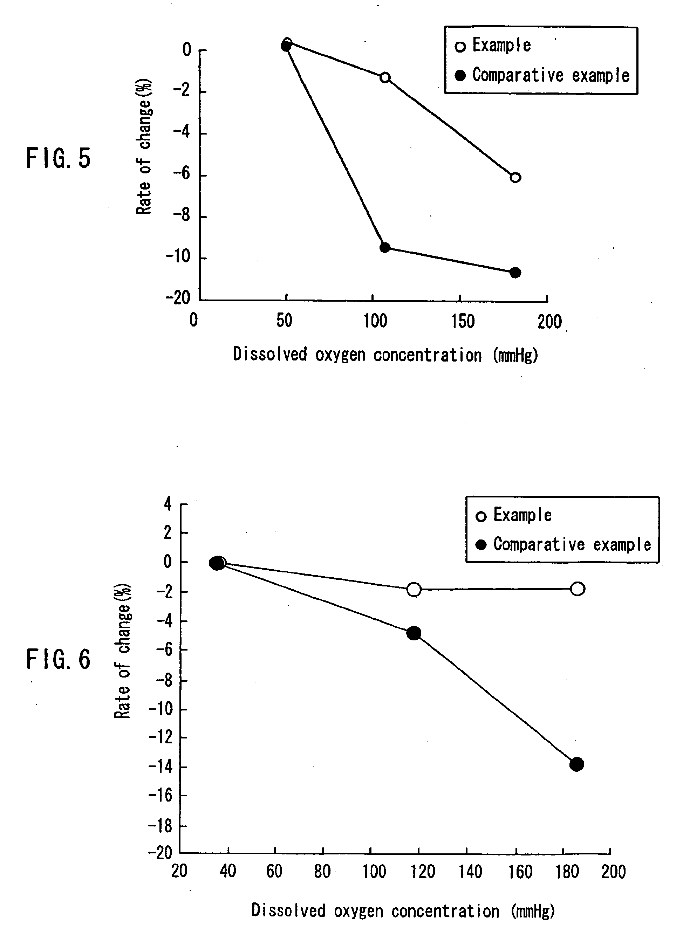

To human whole blood, glucose was added so as to prepare samples with a glucose concentration of 600 mg/ 100 ml. Each of the samples was dropped on the glucose sensor of Example 6 after the start of voltage application (500 mV) between the electrodes of the glucose sensor, and the response current was measured after a lapse of 5 seconds from the dropping of the sample. The same measurement also was carried out using the glucose sensor of Comparative Example 6. With regard to the glucose sensors of Example 6 and Comparative Example 6, the rate of change (%) in the response current value obtained in the measurement of each of the samples with the adjusted dissolved oxygen concentrations (106.4 mmHg and 180.4 mmHg) relative to the response current value obtained in the measurement of the sample with the unadjusted dissolved oxygen concentration (48.9 mmHg) was determined using the above formula (10), as in Example 5. The results are shown in FIG. 5. FIG. 5 is a graph showing the relationship between the concentration of dissolved oxygen in the sample and the rate of change (%). In FIG. 5, o indicates the results obtained in Example 6, and • indicates the results obtained in Comparative Example 6.

As shown in FIG. 5, the glucose sensor of Example 6 using GOD as the oxidoreductase also is less susceptible to the influence of dissolved oxygen, because the deterioration of the sensitivity of this glucose sensor due to dissolved oxygen is smaller than that of the glucose sensor according to Comparative Example 6.

Example 7

In Example 7, the glucose sensor produced in Example 1 was used. In this glucose sensor, CHAPS (ampholytic surfactant) was used as a surfactant and an ACES buffer solution was used as an amine buffer. On the other hand, as a glucose sensor according to Comparative Example 7, a glucose sensor was produced in the same manner as in Example 1, except that cholic acid (an anionic surfactant) was used as a surfactant instead of CHAPS and sodium phosphate was used as a buffer instead of ACES.

To human whole blood, glucose was added so as to prepare samples with a glucose concentration of 100 mg/ 100 ml. The amounts of dissolved oxygen in these samples were adjusted so as to be in the vicinity of 34.7 mmHg (unadjusted), 117.1 mmHg, and 185.3 mmHg, respectively. The adjustment to higher dissolved oxygen concentrations was achieved by mixing the sample having the unadjusted dissolved oxygen concentration with oxygen in a test tube, as in Example 5.

Then, each of the samples was dropped on the respective glucose sensors after the start of voltage application (200 mV) between the electrodes of each glucose sensor. The response current was measured after a lapse of 5 seconds from the dropping of the sample. With regard to this glucose sensor, the rate of change (%) in the response current value obtained in the measurement of each of the samples with the adjusted dissolved oxygen concentrations relative to the response current value obtained in the measurement of the sample with the unadjusted dissolved oxygen concentration was determined using the above formula (10). Also, with regard to the glucose sensor of Comparative Example 7, the measurement and the determination of the rate of change (%) were carried out in the same manner as in Example 7. The results are shown in FIG. 6. FIG. 6 is a graph showing the relationship between the concentration of dissolved oxygen in the sample and the rate of change (%). In FIG. 6, o indicates the results obtained in Example 7, and • indicates the results obtained in Comparative Example 7.

As shown in FIG. 6, the glucose sensor of Comparative Example 7, which used an anionic surfactant instead of an ampholytic surfactant and sodium phosphate as a buffer instead of an amine buffer, was much more susceptible to the influence of dissolved oxygen as compared with the glucose sensor of Example 7, although the glucose sensor of Comparative Example 7 contained smectite. This demonstrates that the influence of oxygen contained in the sample can be prevented by forming an inorganic gel layer containing smectite in the presence of an ampholytic surfactant and an amine buffer as in Example 7. Moreover, in the glucose sensor of Comparative Example 7 using an anionic surfactant, the dispersion could not be disposed on the electrodes easily, which made the production of the glucose sensor itself difficult.

Example 8

A biosensor was produced in the same manner as in Example 1, except that the inorganic gel layer-forming solution was prepared in the following manner.

0.6 g of a synthesized smectite (product name: LUCENTITE SWN, CO-OP CHEMICAL Co. Ltd.), 1 g of a 10% (w/v) aqueous solution of CHAPS (Dojindo Laboratories), and 98.4 g of purified water were mixed together to prepare a smectite suspension (100 g in total), and the smectite suspension was stirred overnight. After the stirring, 2N HCl (4g) was added to 40g of the suspension, and the mixture was stirred overnight (about 24 hours) (the agitator used: a product named "Magnetic Stirrer HS-3E", Iuchi Seieido Co., Ltd.). Although the suspension became cloudy by the addition of HCl, it became transparent after being stirred overnight. A 200 mM succinic acid-sodium acetate buffer solution (pH 4.5) was mixed with this suspension so that the ratio of the buffer solution to the suspension became 1 : 1. After the buffer solution had been added, the suspension had a pH of about 4.5. To the suspension to which the buffer had been added, $[Ru(NH_3)_6]Cl_3$ (Aldrich) further was added as a mediator so that its concentration became 5% (w/v). The resultant mixture was used as an inorganic gel layer-forming solution. The final concentrations of the respective constituents in this inorganic gel layer-forming solution are shown below.

| | |
|---|---|
| LUCENTITE SWN | 0.3% (w/v) |
| CHAPS | 0.3% (w/v) |
| Succinic acid-sodium acetate buffer solution (pH4.5) | 100 mM |
| $[Ru(NH_3)_6]Cl_3$ | 5.0% (w/v) |

Industrial Applicability

As specifically described above, a method for producing a biosensor according to the present invention can provide, for example, a biosensor that can prevent a reduced mediator for indirectly measuring an analyte in a sample from being reoxidized by, for example, oxygen present in the measurement atmosphere, dissolved oxygen in a sample, or the like. Such a biosensor remedies the measurement error caused by the reoxidation of the reduced mediator and thus can achieve excellent measurement accuracy.

**Claims**

1. A method for producing a biosensor, the method comprising:

   providing a substrate having an electrode; and
   forming an inorganic gel layer that contains at least a mediator, an ampholytic surfactant, a buffer, and a layered inorganic compound on a surface of the electrode.

2. The method according to claim 1, wherein the ampholytic surfactant has a positive charge and a negative charge in a single molecule.

3. The method according to claim 2, wherein the ampholytic surfactant is at least one surfactant selected from the group consisting of alkylaminocarboxylate, carboxybetaines, sulfobetaines, and phosphobetaines.

4. The method according to claim 1, wherein the ampholytic surfactant has a positive charge and a negative charge that are separated from each other in a single molecule.

5. The method according to claim 4, wherein the ampholytic surfactant is at least one surfactant selected from the group consisting of carboxybetaines, sulfobetaines, and phosphobetaines.

6. The method according to claim 1, wherein the ampholytic surfactant is alkyldimethylamino acetic acid betaine.

7. The method according to claim 1, wherein the ampholytic surfactant is at least one sulfobetaine selected from the group consisting of CHAPS, CHAPSO, and alkyl hydroxysulfobetaine.

8. The method according to claim 1, wherein the buffer is an amine buffer.

9. The method according to claim 8, wherein the amine buffer is at least one substance selected from the group consisting of Tris, ACES, CHES, CAPSO, TAPS, CAPS, Bis-Tris, TAPSO, TES, Tricine, and ADA.

10. The method according to claim 1, wherein the buffer is a buffer having a carboxyl group.

11. The method according to claim 10, wherein the buffer having a carboxyl group is at least one buffer selected from the group consisting of an acetic acid-sodium acetate buffer, a malic acid-sodium acetate buffer, a malonic acid-sodium acetate buffer, and a succinic acid-sodium acetate buffer.

12. The method according to claim 1, wherein the inorganic gel layer is formed by applying a dispersion containing at least the mediator, the surfactant, the buffer, and the layered inorganic compound.

13. The method according to claim 12, wherein the dispersion is prepared by dispersing the surfactant and the layered inorganic compound in a dispersion medium and then adding the buffer and the mediator to the dispersion in this order.

14. The method according to claim 13, wherein the dispersion is prepared by dispersing the layered inorganic compound in the dispersion medium and then adding the surfactant, an amine buffer, and the mediator to the dispersion in this order.

15. The method according to claim 14, wherein the mediator is at least one substance selected from the group consisting of potassium ferricyanide, cytochrome c, PQQ, $NAD^+$, $NADP^+$, copper complexes, and ruthenium complexes.

16. The method according to claim 14, wherein a pH of the dispersion after the amine buffer has been added is in a range from 5 to 9.

17. The method according to claim 13, wherein the dispersion is prepared by dispersing the layered inorganic compound in the dispersion medium, stirring the dispersion under a strongly acidic condition, and then adding the surfactant, a buffer having a carboxyl group, and the mediator to the dispersion in this order.

18. The method according to claim 17, wherein the mediator is at least one substance selected from the group consisting of ruthenium complexes, osmium complexes, ferrocene, phenazine methosulfate, indophenol, and methylene blue.

19. The method according to claim 17, wherein the strongly acidic condition is a pH in a range from 1 to 3.

20. The method according to claim 17, wherein a pH of the dispersion after the buffer having a carboxyl group has been added is in a range from 3 to 6.

21. The method according to claim 1, wherein the inorganic gel layer is a layer for preventing natural oxidation of the mediator.

22. The method according to claim 1, wherein a layer containing an oxidoreductase further is formed on the inorganic gel layer.

23. The method according to claim 12, wherein the dispersion further contains an oxidoreductase so that the inorganic gel layer containing the oxidoreductase is formed.

24. The method according to claim 1, wherein the layered inorganic compound is a layered clay mineral.

25. The method according to claim 24, wherein the layered clay mineral is an expansive layered clay mineral.

26. The method according to claim 12, wherein the layered inorganic compound and the surfactant are contained in the dispersion so that 1 to 200 mmol of the surfactant is present with respect to 0.3 g of the layered inorganic compound.

27. The method according to claim 12, wherein the layered inorganic compound and the buffer are contained in the dispersion so that 1 to 1000 mM of the buffer is present with respect to 0.3 g of the layered inorganic compound.

28. The method according to claim 22 or 23, wherein the oxidoreductase is at least one enzyme selected from the group consisting of glucose oxidase (GOD), pyranose oxidase, glucose dehydrogenase (GDH), lactate oxidase, lactate dehydrogenase, fructose dehydrogenase, galactose oxidase, cholesterol oxidase, cholesterol dehydrogenase, alcohol oxidase, alcohol dehydrogenase, bilirubin oxidase, glucose-6-phosphate dehydrogenase, amino-acid dehydrogenase, formate dehydrogenase, glycerol dehydrogenase, acyl-CoA oxidase, choline oxidase, 4-hydroxybenzoic acid hydroxylase, maleate dehydrogenase, sarcosine oxidase, and uricase.

29. The method according to claim 1, wherein the mediator is at least one substance selected from the group consisting of potassium ferricyanide, p-benzoquinone and derivatives thereof, indophenol derivatives, β-naphthoquinone-4-sulfonic acid potassium salt, ferrocene derivatives, osmium complexes, ruthenium complexes, $NAD^+$, $NADP^+$, pyrrolo-quinoline quinine (PQQ), methylene blue, cytochrome c, cytochrome b, and copper complexes.

**30.** A biosensor produced by the method according to claim 1.

**Patentansprüche**

**1.** Verfahren zur Herstellung eines Biosensors, wobei man
ein Substrat bereitstellt, das eine Elektrode aufweist; und
eine anorganische Gelschicht, die wenigstens einen Mediator, ein ampholytisches grenzflächenaktives Mittel, einen Puffer, und eine geschichtete anorganische Verbindung enthält, auf einer Oberfläche der Elektrode bildet.

**2.** Verfahren nach Anspruch 1, wobei das ampholytische grenzflächenaktive Mittel eine positive Ladung und eine negative Ladung in einem einzelnen Molekül aufweist.

**3.** Verfahren nach Anspruch 2, wobei das ampholytische grenzflächenaktive Mittel wenigstens ein grenzflächenaktives Mittel ist, das ausgewählt ist unter Alkylaminocarboxylat, Carboxybetainen, Sulfobetainen und Phosphobetainen.

**4.** Verfahren nach Anspruch 1, wobei das ampholytische grenzflächenaktive Mittel eine positive Ladung und eine negative Ladung aufweist, die in einem einzelnen Molekül voneinander getrennt sind.

**5.** Verfahren nach Anspruch 4, wobei das ampholytische grenzflächenaktive Mittel wenigstens ein grenzflächenaktives Mittel ist, das ausgewählt ist unter Carboxybetainen, Sulfobetainen und Phosphobetainen.

**6.** Verfahren nach Anspruch 1, wobei das ampholytische grenzflächenaktive Mittel Alkyldimethylaminoessigsäure-Betain ist.

**7.** Verfahren nach Anspruch 1, wobei das ampholytische grenzflächenaktive Mittel wenigstens ein Sulfobetain ist, das ausgewählt ist unter CHAPS, CHAPSO und Alkylhydroxysulfobetain.

**8.** Verfahren nach Anspruch 1, wobei der Puffer ein Aminpuffer ist.

**9.** Verfahren nach Anspruch 8, wobei der Aminpuffer wenigstens eine Substanz ist, die ausgewählt ist unter Tris, ACES, CHES, CAPSO, TAPS, CAPS, Bis-Tris, TAPSO, TES, Tricin und ADA.

**10.** Verfahren nach Anspruch 1, wobei der Puffer ein Puffer ist, der eine Carboxylgruppe aufweist.

**11.** Verfahren nach Anspruch 10, wobei der Puffer, der eine Carboxylgruppe aufweist, wenigstens ein Puffer ist, der ausgewählt ist unter einem Essigsäure-Natriumacetat-Puffer, einem Äpfelsäure-Natriumacetat-Puffer, einem Malonsäure-Natriumacetat-Puffer und einem Bernsteinsäure-Natriumacetat-Puffer.

**12.** Verfahren nach Anspruch 1, wobei die anorganische Gelschicht gebildet wird durch Auftragen einer Dispersion, die wenigstens den Mediator, das grenzflächenaktive Mittel, den Puffer und die geschichtete anorganische Verbindung enthält.

**13.** Verfahren nach Anspruch 12, wobei die Dispersion hergestellt wird, indem man das grenzflächenaktive Mittel und die geschichtete anorganische Verbindung in einem Dispergiermedium dispergiert und anschließend den Puffer und den Mediator der Dispersion in dieser Reihenfolge zugibt.

**14.** Verfahren nach Anspruch 13, wobei die Dispersion hergestellt wird, indem man die geschichtete anorganische Verbindung in dem Dispergiermedium dispergiert und anschließend das grenzflächenaktive Mittel, einen Aminpuffer und den Mediator der Dispersion in dieser Reihenfolge zugibt.

**15.** Verfahren nach Anspruch 14, wobei der Mediator wenigstens eine Substanz ist, die ausgewählt ist unter Kaliumferricyanid, Cytochrom c, PQQ, $NAD^+$, $NADP^+$, Kupfer-Komplexen und Ruthenium-Komplexen.

**16.** Verfahren nach Anspruch 14, wobei der pH der Dispersion nach Zugabe des Aminpuffers in einem Bereich von 5 bis 9 liegt.

**17.** Verfahren nach Anspruch 13, wobei die Dispersion hergestellt wird, indem man die geschichtete anorganische Verbindung in dem Dispergiermedium dispergiert, die Dispersion unter stark sauren Bedingungen rührt, und anschließend das grenzflächenaktive Mittel, einen Puffer, der eine Carboxylgruppe aufweist, und den Mediator der Dispersion in dieser Reihenfolge zugibt.

**18.** Verfahren nach Anspruch 17, wobei der Mediator wenigstens eine Substanz ist, die ausgewählt ist unter Ruthenium-Komplexen, Osmium-Komplexen, Ferrocen, Phenazinmethosulfat, Indophenol und Methylenblau.

**19.** Verfahren nach Anspruch 17, wobei die stark saure Bedingung ein pH-Wert in einem Bereich von 1 bis 3 ist.

**20.** Verfahren nach Anspruch 17, wobei der pH-Wert der Dispersion nach Zugabe des eine Carboxylgruppe aufweisenden Puffers in einem Bereich von 3 bis 6 liegt.

**21.** Verfahren nach Anspruch 1, wobei die anorganische Gelschicht eine Schicht zur Verhinderung natürlicher Oxidation des Mediators ist.

**22.** Verfahren nach Anspruch 1, wobei weiterhin eine Schicht, die eine Oxidoreduktase enthält, auf der anorganischen Gelschicht gebildet wird.

**23.** Verfahren nach Anspruch 12, wobei die Dispersion weiterhin eine Oxidoreduktase enthält, so dass die anorganische Gelschicht, die die Oxidoreduktase enthält, gebildet wird.

**24.** Verfahren nach Anspruch 1, wobei die geschichtete anorganische Verbindung ein geschichtetes Tonmineral ist.

**25.** Verfahren nach Anspruch 24, wobei das geschichtete Tonmineral ein expansives geschichtetes Tonmineral ist.

**26.** Verfahren nach Anspruch 12, wobei die geschichtete anorganische Verbindung und das grenzflächenaktive Mittel in der Dispersion derart enthalten sind, dass 1 bis 200 mMol des grenzflächenaktiven Mittels bezogen auf 0,3 g der geschichteten anorganischen Verbindung vorliegen.

**27.** Verfahren nach Anspruch 12, wobei die geschichtete anorganische Verbindung und der Puffer in der Dispersion derart enthalten sind, dass 1 bis 1000 mM des Puffers bezogen auf 0,3 g der geschichteten anorganischen Verbindung vorliegen.

**28.** Verfahren nach Anspruch 22 oder 23, wobei die Oxidoreduktase wenigstens ein Enzym ist, das ausgewählt ist unter Glukose-Oxidase (GOD), Pyranose-Oxidase, Glukose-Dehydrogenase (GDH), Laktat-Oxidase, Laktat-Dehydrogenase, Fruktose-Dehydrogenase, Galaktose-Oxidase, Cholesterin-Oxidase, Cholesterin-Dehydrogenase, Alkohol-Oxidase, Alkohol-Dehydrogenase, Bilirubin-Oxidase, Glukose-6-phosphat-Dehydrogenase, Aminosäure-Dehydrogenase, Formiat-Dehydrogenase, Glycerin-Dehydrogenase, Acyl-CoA-Oxidase, Cholin-Oxidase, 4-Hydroxybenzoesäure-Hydroxylase, Maleat-Dehydrogenase, Sarcosin-Oxidase und Uricase.

**29.** Verfahren nach Anspruch 1, wobei der Mediator wenigstens eine Substanz ist, die ausgewählt ist unter Kaliumferricyanid, p-Benzochinon und Derivaten davon, Indophenol-Derivaten, β-Naphthochinon-4-sulfonsäure-Kaliumsalz, Ferrocen-Derivaten, Osmium-Komplexen, Ruthenium-Komplexen, $NAD^+$, $NADPH^+$, Pyrrolo-chinolin-chinin (PQQ), Methylenblau, Cytochrom c, Cytochrom b und Kupfer-Komplexen.

**30.** Biosensor, hergestellt nach dem Verfahren nach Anspruch 1.

**Revendications**

**1.** Procédé pour produire un biocapteur, procédé comprenant
la fourniture d'un substrat comportant une électrode ; et la formation d'une couche de gel inorganique contenant au moins un médiateur, un agent de surface ampholyte, un tampon, et un composé inorganique stratifié sur la surface de l'électrode.

**EP 1 679 508 B1**

2. Procédé selon la revendication 1, dans lequel l'agent de surface ampholyte présente une charge positive et une charge négative dans une seule molécule.

3. Procédé selon la revendication 2, dans lequel l'agent de surface ampholyte est au moins un agent de surface choisi dans le groupe comprenant l'alkylaminocarboxylate, les carboxybétaïnes, les sulfobétaïnes et les phosphobétaïnes.

4. Procédé selon la revendication 1, dans lequel l'agent du surface ampholyte présente une charge positive et une charge négative séparées l'une de l'autre dans une seule molécule.

5. Procédé selon la revendication 4, dans lequel l'agent de surface ampholyte est au moins un agent de surface choisi dans le groupe comprenant les carboxybétaïnes, les sulfobétaïnes et les phosphobétaïnes.

6. Procédé selon la revendication 1, dans lequel l'agent de surface ampholyte est une alkyl diméthylamino acétique acide bétaïne.

7. Procédé selon la revendication 1, dans lequel l'agent de surface ampholyte est au moins une sulfobétaïne choisie dans le groupe composé des CHAPS, CHAPSO, et de l'alkyl hydroxysulfobétaïne.

8. Procédé selon la revendication 1, dans lequel le tampon est un tampon amine.

9. Procédé selon la revendication 8, dans lequel le tampon amine est au moins une substance choisie dans le groupe composé des Tris, ACES, CHES, CAPSO, TAPS, CAPS, Bis-Tris, TAPSO, TES, Tricine, et ADA.

10. Procédé selon la revendication 1, dans lequel le tampon est un tampon du groupe carboxyle.

11. Procédé selon la revendication 10, dans lequel le tampon du groupe carboxyle est au moins un tampon choisi dans le groupe composé d'un tampon d'acétate sodique-acide acétique, un tampon d'acétate sodique-acide malique, un tampon d'acétate sodique-acide malonique, et un tampon d'acétate sodique-acide succinique.

12. Procédé selon la revendication 1, dans lequel la couche de gel inorganique est formée en appliquant une dispersion contenant au moins le médiateur, l'agent de surface, le tampon et le composé inorganique stratifié.

13. Procédé selon la revendication 12, dans lequel la dispersion est préparée en dispersant l'agent de surface et le composé inorganique stratifié dans un milieu de dispersion puis en ajoutant le tampon et le médiateur à la dispersion, en respectant cet ordre.

14. Procédé selon la revendication 13, dans lequel la dispersion est préparée en dispersant le composé inorganique stratifié dans le milieu de dispersion et en ajoutant ensuite l'agent de surface, un tampon amine et le médiateur à la dispersion, en respectant cet ordre.

15. Procédé selon la revendication 14, dans lequel le médiateur est au moins une substance choisie dans le groupe composé de ferrocyanide de potassium, de cytochrome c, PQQ, NAD+, NADP+, de complexes de cuivre et de complexes de ruthénium.

16. Procédé selon la revendication 14, dans lequel le pH de la dispersion après adjonction du tampon amine se situe dans une fourchette allant de 5 à 9.

17. Procédé selon la revendication 13, dans lequel la dispersion est préparée en dispersant le composé inorganique stratifié dans le milieu de dispersion, en remuant la dispersion dans des conditions fortement acides, puis en ajoutant à la dispersion l'agent de surface, un tampon du groupe carboxyle, et le médiateur, en respectant cet ordre.

18. Procédé selon la revendication 17, dans lequel le médiateur est au moins une substance choisie dans le groupe composé de complexes de ruthénium, de complexes d'osmium, de ferrocène, de phénazine, de méthosulfate, d'indophénol et de bleu de méthylène.

19. Procédé selon la revendication 17, dans lequel la condition fortement acide correspond à un pH allant de 1 à 3.

20. Procédé selon la revendication 17, dans lequel le pH de la dispersion après adjonction du tampon du groupe

21

carboxyle se situe dans une fourchette allant de 3 à 6.

21. Procédé selon la revendication 1, dans lequel la couche de gel inorganique est une couche destinée à empêcher l'oxydation naturelle du médiateur.

22. Procédé selon la revendication 1, dans lequel une couche contenant une oxydoréductase est ensuite formée sur la couche de gel inorganique.

23. Procédé selon la revendication 12, dans lequel la dispersion comprend également une oxydoréductase de façon à permettre la formation de la couche de gel inorganique contenant l'oxydoréductase.

24. Procédé selon la revendication 1, dans lequel le composé inorganique stratifié est un minéral argileux stratifié.

25. Procédé selon la revendication 24, dans lequel le composé inorganique stratifié est un minéral argileux stratifié gonflant.

26. Procédé selon la revendication 12, dans lequel le composé inorganique stratifié et l'agent de surface sont contenus dans la dispersion de façon à obtenir une proportion de 1 à 200 mmol d'agent de surface pour 0,3 g de composé inorganique stratifié.

27. Procédé selon la revendication 12, dans lequel le composé inorganique stratifié et le tampon sont contenus dans la dispersion de façon à obtenir une proportion de 1 à 1000 mM de tampon pour 0,3 g de composé inorganique stratifié.

28. Procédé selon les revendications 22 ou 23, dans lequel l'oxydoréductase est au moins une enzyme choisie dans le groupe composé de glucose-oxydase (GOD), de pyranose-oxydase, de glucose-déshydrogénase,(GHD), de lactate-oxydase, de lactate-déshydrogénase, de fructose-déshydrogénase, de galactose-oxydase, de cholestérol-oxydase, de cholestérol-déshydrogénase, d'alcool-oxydase, d'alcool-déshydrogénase, de bilirubine-oxydase, de glucose-6-phosphate-déshydrogénase, d'aminoacide-déshydrogénase, de formiate-déshydrogénase, de glycérol-déshydrogénase, d'acyl-CoA-oxydase, de choline-oxydase, d'acide 4-hydroxybenzoïque hydroxylase, de maléate-déshydrogénase, de sarcosine-oxydase, et d'uricase.

29. Procédé selon la revendication 1, dans lequel le médiateur est au moins une substance choisie dans le groupe composé de ferrocyanide de potassium, de p-benzoquinone et de ses dérivés, de dérivés d'indophénol, de sel de potassium d'acide sulfonique 4-β-naphthoquinone, de dérivés de ferrocène, de complexes d'osmium, de complexes de ruthénium, de NAD+, de NADP+, de pyrrolo-quinoline quinine (PQQ), de bleu de méthylène, de cytochrome c, de cytochrome b, et de complexes de cuivre.

30. Biocapteur produit en utilisant le procédé décrit dans la revendication 1.

FIG. 1A

FIG. 1B

FIG. 1C

FIG. 1D

FIG. 1E

FIG. 1F

FIG. 2

FIG. 3A

FIG. 3B

25

FIG. 4

FIG. 5

FIG. 6

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 1291153 A
- EP 0884392 A
- EP 1235068 A
- EP 1336839 A

**Non-patent literature cited in the description**

- **D. W, Thompson ; J. T. Butterworth.** *J. Colloid Interf. Sci.,* 1992, vol. 151, 236-243